(19) Europäisches Patentamt
European Patent Office
Office européen des brevets

(11) Veröffentlichungsnummer: **0 158 115**
**B1**

(12) # EUROPÄISCHE PATENTSCHRIFT

(45) Veröffentlichungstag der Patentschrift: **31.05.89**

(51) Int. Cl.⁴: **C 07 D 223/22**

(21) Anmeldenummer: **85102501.5**

(22) Anmeldetag: **06.03.85**

(54) **Verfahren zur Herstellung von Iminodibenzyl.**

(30) Priorität: **10.04.84 DE 3413496**

(43) Veröffentlichungstag der Anmeldung: **16.10.85 Patentblatt 85/42**

(45) Bekanntmachung des Hinweises auf die Patenterteilung: **31.05.89 Patentblatt 89/22**

(84) Benannte Vertragsstaaten: **AT CH DE FR GB IT LI NL**

(56) Entgegenhaltungen:
**CH-A-331 207**
**US-A-2 800 470**

(73) Patentinhaber: **CL PHARMA AKTIENGESELLSCHAFT, St. Peter- Strasse 25, A-4021 Linz (AT)**

(72) Erfinder: **Raml, Walter, Dr., A-4202 Hellmonsödt 246 (AT)**
Erfinder: **Stern, Gerhard, Dr. Dipl.- Ing., Unterrudersbach 7, A-4180 Sonnberg i.M. (AT)**
Erfinder: **Saischek, Gerald, Dipl.- Ing., Roseggerstrasse 4, A-4600 Wels (AT)**

(74) Vertreter: **Kunz, Ekkehard, Dr., Chemie Holding AG Patentwesen St. Peter- Strasse 25, A-4021 Linz (AT)**

**EP 0 158 115 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft ein Verfahren zur Herstellung von Iminodibenzyl durch Umsetzung von geschmolzenem 2,2'-Diaminodibenzyl und Phosphorsäuren in kontinuierlicher Fahrweise.

Iminodibenzyl und seine Derivate stellen wichtige Ausgangssubstanzen für die Synthese von Arzneimitteln mit breitem pharmazeutischen Anwendungsbereich dar. Die Herstellung von Iminodibenzyl erfolgte in der Praxis meist durch Erhitzen von Diphosphaten des 2,2'-Diaminodibenzyls in geschmolzenem Zustand auf etwa 250° - 320°C (CH-PS 331 207), bei einer Reaktionszeit von 40 Minuten, wobei eine Ausbeute von 92 % erhalten wird. Die Gewinnung des Iminodibenzyls aus der Reaktionsmischung erfolgt dann durch Auskochen mit Benzol und Abdampfen des Lösungsmittels nach Waschen der benzolischen Phase.

In der US-A-2 800 470 werden halogensubstituierte Iminodibenzylderivate und deren Herstellungsmethode beschrieben. Das betreffende Verfahren ist diskontinuierlich und sieht einen zweistufigen Reaktionsablauf für die Bildung und Weiterverwendung des Diphosphats vor, wobei in der ersten Stufe mit Ortho- und in der zweiten mit Polyphosphorsäure gearbeitet wird. In der AT-PS 200 579 ist ferner allgemein erwähnt, daß Iminodibenzyl auch ausgehend von freiem 2,2'-Diaminodibenzyl durch Erhitzen mit Polyphosphorsäure erhältlich ist, wobei aber nähere Verfahrensbedingungen und Ausbeuten nicht angegeben werden.

Alle diese bekannten Verfahren sind diskontinuierlich, und daher zeitaufwendig, wobei die komplizierte Aufarbeitung zusätzlich eine wirtschaftliche Belastung darstellt. Geht man vom Diphosphat des Diaminodibenzyls aus, so stellt das der Reaktion vorausgehende Aufschmelzen des bei 266 - 268°C schmelzenden Diphosphats eine besonders energieaufwendige Maßnahme dar.

Es konnte nun gefunden werden, daß man Iminodibenzyl ausgehend von 2,2'-Diaminodibenzyl in vorteilhafter und energiesparender Weise kontinuierlich herstellen und reines Iminodibenzyl in einer einfachen Aufarbeitungsstufe direkt erhalten kann, wenn man auf nicht mehr als 200°C vorgewärmtes Diaminodibenzyl und auf etwa der gleichen Temperatur befindliche Phosphor-bzw. Polyphosphorsäure getrennt in die erste Stufe einer mehrstufigen Reaktion einspeist, wobei die Neutralisationswärme zur Erreichung der Reaktionstemperatur herangezogen wird und die Aufarbeitung durch einfache Phasentrennung des heißen Reaktionsproduktes bewerkstelligt wird.

Gegenstand der vorliegenden Erfindung ist demnach ein Verfahren zur Herstellung von Iminodibenzyl der Formel

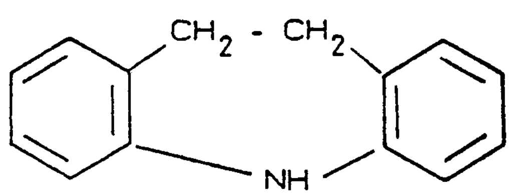

I

durch Erhitzen von 2,2'-Diaminodibenzyl der Formel

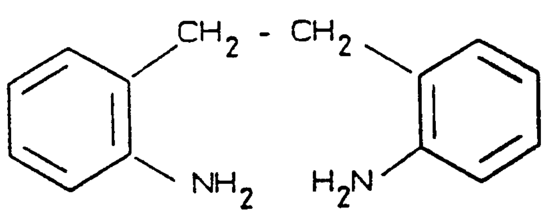

II

mit Phosphorsäuren in geschmolzenem Zustand, das dadurch gekennzeichnet ist, daß man die Reaktion kontinuierlich in mehreren Stufen durchführt wobei

a) geschmolzenes 2,2'-Diaminodibenzyl und eine Phosphorsäure, die je eine Temperatur zwischen 160 und 200°C besitzen, im Molverhältnis von Diaminodibenzyl: $P_2O_5$ von 1 : 1 bis 1 : 2,5 getrennt in die erste Stufe eingespeist werden und dort unter Wärmeentwicklung die Neutralisation erfolgt und man in den anschließenden Reaktionsstufen die Kondensation bei einer Temperatur von 260°C bis 320°C durchführt, worauf man

b) das Reaktionsgemisch ungekühlt aus der letzten Stufe in kontinuierlichem Abfluß in einen geheizten Separator überführt und bei 160°C bis 300°C in einen die obere Phase aus geschmolzenem Iminodibenzyl enthaltenden Produktstrom und in einen als untere Phase Phosphorsäure und Ammonphosphate enthaltenden Abstrom auftrennt.

Einer der wesentlichen Vorteile des erfindungsgemäßen Verfahrens liegt darin, daß die bei der Reaktion von 2,2'-Diaminodibenzyl mit der Phosphorsäure in der ersten Stufe freiwerdende Neutralisationswärme zur weiteren Erwärmung auf die erforderliche Reaktionstemperatur benützt wird. Dadurch genügt es, die beiden Reaktionskomponenten auf eine wesentlich niedrigere Temperatur und zwar nur auf 160 - 200°C, vorzuwärmen,

als dies im Fall des Diphosphats von 2,2'-Diaminodibenzyl geschehen muß, welches mindestens bis zu seinem Schmelzpunkt von 266 - 268°C erwärmt werden muß. Außerdem kann im Falle der Verwendung des Diphosphats als Ausgangssubstanz die Neutralisationswärme nicht genützt werden, da diese bei der Isolierung des Diphosphats verloren geht. Daraus resultiert für das vorliegende Verfahren eine besonders vorteilhafte Energiebilanz, wobei die Energieeinsparung ca. 50 % der insgesamt für die Reaktion benötigten Energie beträgt, sodaß sich das Verfahren auch durch eine außergewöhnlich gute Wirtschaftlichkeit auszeichnet.

Eine optimale Ausnutzung der freiwerdenden Neutralisationswärme für die Gesamtreaktion ist bei dem erfindungsgemäßen Verfahren dadurch gewährleistet, daß die Reaktionspartner getrennt und kontinuierlich in den Reaktionsraum dosiert werden. Dadurch ist es im Gegensatz zu den diskontinuierlichen Verfahren, bei denen eine gleichmäßige Wärmezuführung von außen nur sehr schwierig gelingt möglich, lokale Überhitzung bei gleichzeitiger vermehrter Bildung von Nebenprodukten zu vermeiden, wodurch deutlich bessere Ausbeuten von bis zu 98,9 % erreicht werden und außerdem das Reaktionsprodukt bereits eine derart gute Reinheit besitzt, daß nach der Abtrennung der Phosphorsäurephase eine weitere Reinigung des Iminodibenzyls nicht mehr nötig ist. Diese Tatsache stellt einen weiteren entscheidenden Vorteil des Verfahrens dar, dadurch den Wegfall der komplizierten Aufarbeitung des Reaktionsgemisches durch Abkühlen der Masse, Extraktion des Iminodibenzyls mit einem Lösungsmittel, Waschen mit Säure und Wasser, Trocknung der Lösung und Abdampfen des Lösungsmittels, wie sie für die diskontinuierlichen Verfahren beschrieben sind, eine sehr einfache und wirtschaftliche Aufarbeitungsmethode gegeben ist, wodurch eine bedeutende Verkürzung der benötigten Gesamtzeit erreicht wird.

Eine zusätzliche Verkürzung der zur Herstellung des Iminodibenzyls benötigten Gesamtzeit ergibt sich weiters aus der Tatsache, daß das Erwärmen und Aufschmelzen der Reaktionskomponenten, das bei den diskontinuierlichen Verfahren ein Mehrfaches der für die eigentliche Umsetzung zum Iminodibenzyl benötigten Zeit erfordert, beim vorliegenden kontinuierlichen Verfahren zeitlich parallel zur Reaktion erfolgt und damit keinen zusätzlichen Zeitaufwand erfordert.

Aufgrund der daraus resultierenden, relativ kurzen Arbeitszeit sowie aufgrund der oben beschriebenen Möglichkeit, durch getrennte kontinuierliche Zuführung der vorerhitzten Einsatzstoffe die Temperaturführung während der Reaktion optimal zu kontrollieren, ist es beim vorliegenden kontinuierlichen Verfahren möglich, durch entsprechende Dimensionierung der Apparatur weitaus größere Raum-Zeit-Ausbeuten zu erreichen, als dies bei den diskontinuierlichen Verfahren, bei denen immer die Gefahr von lokalen Überhitzungen besteht, möglich ist.

Das als Ausgangssubstanz eingesetzte 2,2'-Diaminodibenzyl wurde analog zur CH-PS 331 207 durch Reduktion von 2,2'-Dinitrodibenzyl mit Raney-Nickel in organischer Lösung hergestellt.

Zur Umsetzung wird wasserfreie Phosphorsäure bzw. Polyphosphorsäure mit einem Gehalt von 72,4 - 84 % Phosphorpentoxid verwendet.

Bei der Durchführung des erfindungsgemäßen Verfahrens ist zu beachten, daß zwar in der ersten Stufe durch die Neutralisation Wärme entsteht, die in den weiteren Stufen stattfindende Kondensation aber endotherm ist, sodaß dort durch entsprechende Wärmezufuhr dafür gesorgt werden muß, daß die nötige Reaktionstemperatur von über 260°C gehalten wird. Besonders vorteilhaft ist es, die in die erste Stufe einzuführenden Reaktionskomponenten soweit innerhalb des erfindungsgemäßen Bereiches von 160 - 200°C vorzuwärmen, daß sich unter Ausnutzung der Neutralisationswärme eine Temperatur einstellt, die über der vorgesehenen Reaktionstemperatur liegt. Dieser Wärmeüberschuß steht dann für die einsetzende endotherme Reaktion zur Verfügung. Besonders bevorzugt ist, daß man in der ersten Stufe eine Temperatur von 290 - 300°C einstellt und für die weiteren Stufen eine Reaktionstemperatur von 280 - 290°C wählt.

Vorteilhafterweise wird das Verfahren in 8 - 15 Stufen durchgeführt, wobei beispielsweise ein Mehrkammerreaktor, dessen Kammern beheizt sind, mit Vorteil eingesetzt werden kann. Bei diesem kann jede Kammer gesondert beheizt werden. Durch die erfindungsgemäße Maßnahme, durch eine höhere Temperatur in der ersten Stufe den erhöhten Wärmebedarf in der oder den nächstfolgenden Stufen zu kompensieren, ist es vorteilhafterweise auch möglich, die Heizung nur zonenweise zu unterteilen, z. B. in zwei Zonen.

Im Verlaufe des Durchgangs durch die nacheinander angeordneten Reaktionskammern erfolgt eine fast vollständige Umsetzung, wodurch extrem hohe Ausbeuten an Iminodibenzyl erhalten werden. In einer besonders bevorzugten Anordnung sind die einzelnen Reaktionskammern übereinander angeordnet, wobei das 2,2'-Diaminodibenzyl und die Phosphorsäure von unten in den Reaktor eingespeist werden und beim weiteren Durchgang durch die gerührten Reaktionskammern im aufsteigenden Strom miteinander reagieren.

Bei der Umsetzung von 2,2'-Diaminodibenzyl mit Phosphorsäure wird bevorzugt ein Molverhältnis von 2,2'-Diaminodibenzyl zu $P_2O_5$ von 1 : 1 bis 1 : 2, besonders bevorzugt von 1 : 1 bis 1 : 1,5 eingesetzt, da bei einem Molverhältnis von unter 1 : 1 die Ausbeute an Iminodibenzyl sinkt. Der in den diskontinuierlichen Verfahren teilweise verwendete Überschuß bis zu einem Molverhältnis von 1 : 7 bringt keine Verbesserung der Ausbeuten und dient dort vor allem als Lösungsmittel für das 2,2'-Diaminodibenzyl-Diphosphat sowie zur Erzielung eines besseren Wärmeübergangs beim Aufschmelzen.

Die Phasentrennung nach erfolgter Umsetzung in eine obere Phase von geschmolzenem Iminodibenzyl und eine untere Phase von Phosphorsäure und Ammonphosphaten wird zweckmäßig bei 160 - 290°C durchgeführt. Eine besonders einwandfreie Trennung gelingt bei Temperaturen von 230 bis 250°C.

Das erfindungsgemäße Verfahren wird durch die folgenden Beispiele näher erläutert:

**Beispiel 1:**

In einem Mehrkammerreaktor mit einem Volumen von 420 ml, der durch Einbauten in 8 Reaktionskammern getrennt ist, werden unter Rühren kontinuierlich 5,03 ml/m in auf 200°C erwärmtes 2,2'-Diaminodibenzyl, sowie 3,24 ml/min wasserfreie auf 200°C erwärmte Phosphorsäure mit einem Gehalt von 72,6 % $P_2O_5$ entsprechend einem Molverhältnis Diaminodibenzyl zu $P_2O_5$ von 1 : 1,25, in die untere Reaktionskammer eingebracht. Die Reaktion erfolgt im aufsteigenden Strom unter Rühren beim Durchgang durch sämtliche Reaktionskammern. Die Temperatur betrug in der unteren Reaktionskammer 292°C, in den oberen Kammern 286°C. Die mittlere Verweilzeit beim Durchgang durch die Apparatur war 46 Minuten. Das Reaktionsgemisch fließt aus der obersten Kammer kontinuierlich in ein geheiztes Trenngefäß ab, in dem es bei Temperaturen von 230 bis 250°C in zwei Phasen aufgetrennt wird. Sowohl die obere Iminodibenzylphase als auch die untere Phosphorsäurephase werden nach der Auftrennung kontinuierlich abgezogen. Nach einer Reaktionszeit von 592 Minuten werden 2 820 g Iminodibenzyl, entsprechend einer Ausbeute von 98,9 % erhalten.

In der folgenden Tabelle sind die Daten der Beispiele 2 bis 6 angegeben, bei denen 2,2'-Diaminodibenzyl mit Phosphorsäure analog zu Beispiel 1 zur Reaktion gebracht wurden.

Die Reinheit des Iminodibenzyls wurde jeweils gaschromatographisch gemessen und lag zwischen 98,9 und 99,7 %, der Schmelzpunktlag bei 105 bis 107°C.

| | Diaminodibenzyl Phosphorsäure (ml/min) | Molverhältnis Diaminodibenzyl: $P_2O_5$ | Verweilzeit (min) | Einsatz-stoffe | Temperatur oben Separator unten (°C) | Gesamtzeit (min.) | Iminodibenzyl g | % Aus-beute |
|---|---|---|---|---|---|---|---|---|
| **Biespiel 2** | 13,1 / 8,6 | 1 : 1,25 | 18 | 180 | 283 / 291 230 - 250 | 657 | 8 040 | 97,4 |
| **Biespiel 3** | 8,72 / 5,43* | 1 : 1,31 | 26 | 160 | 263 / 296 160 - 190 | 655 | 4 865 | 95,5 |
| **Biespiel 4** | 9,21 / 5,06 | 1 : 1,05 | 26 | 190 | 283 / 296 210 - 240 | 649 | 4 370 | 90,7 |
| **Biespiel 5** | 8,98 / 7,77** | 1 : 2,14 | 26 | 170 | 271 / 290 230 - 250 | 594 | 4 615 | 96,9 |
| **Biespiel 6** | 12,98 / 4,88 | 1 : 0,75 | 18 | 200 | 286 / 292 250 - 290 | 660 | 6 623 | 81,0 |

* Phosphorsäure mit 76 % $P_2O_5$

** Phosphorsäure mit 84 % $P_2O_5$

*** Beispiel 6 gilt nicht als representativ u. illustriert nicht die Erfindung; es soll nur verdeutlichen, daß ein Mindestverhältnis von 1 : 1 essentiell ist.

**Patentansprüche**

1. Verfahren zur Herstellung von Iminodibenzyl der Formel (I)

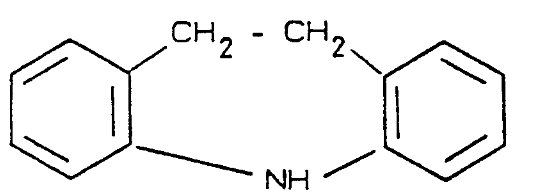

I

durch Erhitzen von 2,2' Diaminodibenzyl der Formel (II)

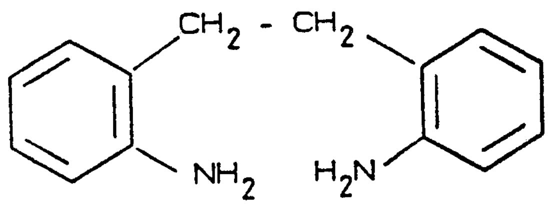

II

mit Phosphorsäuren in geschmolzenem Zustand, dadurch gekennzeichnet, daß man die Reaktion kontinuierlich in mehreren Stufen durchführt, wobei

a) geschmolzenes 2,2'-Diaminodibenzyl und eine Phosphorsäure, die je eine Temperatur zwischen 160 und 200°C besitzen, im Molverhältnis von Diaminodibenzyl: $P_2O_5$ von 1 : 1 bis 1 : 2,5 getrennt in die erste Stufe eingespeist werden und dort unter Wärmeentwicklung die Neutralisation erfolgt und man in den anschließenden Reaktionsstufen die Kondensation bei einer Temperatur von 260°C bis 320°C durchführt, worauf man

b) das Reaktionsgemisch ungekühlt aus der letzten Stufe in kontinuierlichem Abfluß in einen geheizten Separator überführt und bei 160°C bis 300°C in einen die obere Phase aus geschmolzenem Iminodibenzyl enthaltenden Produktstrom und in einen als untere Phase Phosphorsäure und Ammonphosphate enthaltenden Abstrom auftrennt.

2. Vefahren nach Anspruch 1, dadurch gekennzeichnet, daß man in der ersten Reaktionsstufe eine Temperatur von 290 bis 300°C einstellen läßt und in den weiteren Reaktionsstufen eine Temperatur von 280 - 290°C eingehalten wird.

3. Verfahren nach den Ansprüchen 1 und 2, dadurch gekennzeichnet, daß die Reaktion in 8 bis 15 Stufen durchgeführt wird.

4. Verfahren nach den Ansprüchen 1 bis 3, dadurch gekennzeichnet, daß man das 2,2'-Diaminodibenzyl und die Phosphorsäure in einem Molverhältnis von Diaminodibenzyl: $P_2O_5$ von 1 : 1 bis 1 : 2 einsetzt.

5. Verfahren nach Anspruch 4, dadurch gekennzeichnet, daß man das 2,2'-Diaminodibenzyl und die Phosphorsäure in einem Molverhältnis von Diaminodibenzyl: $P_2O_5$ von 1 : 1 bis 1 : 1,5 einsetzt.

6. Verfahren nach den Ansprüchen 1 bis 5, dadurch gekennzeichnet, daß man die Trennung in Produktstrom und Abstrom bei einer Temperatur von 230 bis 250°C vornimmt.

## Claims

1. Process for the preparation of iminodibenzyl of the formula (I)

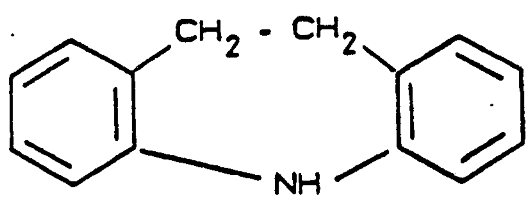

I

by heating 2,2'-diaminodibenzyl of the formula (II)

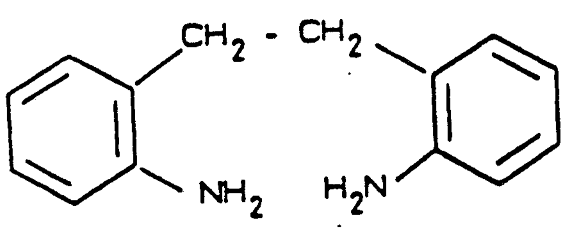

II

with phosphoric acids in the molten state, characterized in that reaction is carried out continuously in several stages, wherein

a) molten 2,2'-diaminodibenzyl and a phosphoric acid, each of which has a temperature of between 160 and 200°C, are fed separately in a molar ratio of diaminodibenzyl: $P_2O_5$ of 1 : 1 to 1 : 2.5 into the first stage, the neutralization takes place there, with evolution of heat, and the condensation is carried out in the subsequent reaction stages at a temperature of 260°C to 320°C, after which

b) the uncooled reaction mixture from the last stage is transferred continuously into a heated separator and is separated at 160° C to 300° C into a product stream containing the upper phase of molten iminodibenzyl and a discharge stream containing, as the lower phase, phosphoric acid and ammonium phosphates.

2. Process according to claim 1, wherein a temperature of 290 to 300° C can be established in the first reaction stage and a temperature of 280 - 290° C is maintained in the subsequent reaction stages.

3. Process according to claims 1 and 2, wherein the reaction is carried out in 8 to 15 stages.

4. Process according to claims 1 to 3, wherein the 2,2'-diaminodibenzyl and the phosphoric acid are employed in a molar ratio of diaminodibenzyl: $P_2O_5$ of 1 : 1 to 1 : 2.

5. Process according to claim 4, wherein the 2,2'-diaminodibenzyl and the phosphoric acid are employed in a molar ratio of diaminodibenzyl: $P_2O_5$ of 1 : 1 to 1 : 1.5.

6. Process according to claims 1 to 5, wherein the separation into the product stream and discharge stream is carried out at a temperature of 230 to 250° C.

**Revendications**

1. Procédé de préparation d'iminodibenzyle répondant à la formule (I):

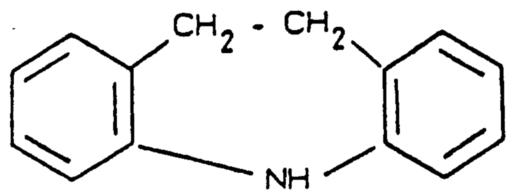

I

par chauffage de 2,2'-diaminodibenzyle répondant à la formule (II):

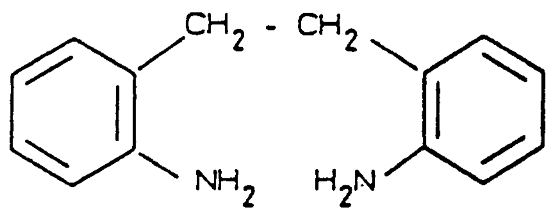

II

avec des acides phosphoriques à l'état fondu, caractérisé en ce qu'on effectue la réaction en continu en plusieurs stades,

a) en introduisant séparément dans le premier stade du 2,2'-diaminodibenzyle fondu et un acide phosphorique, présentant chacun une température comprise entre 160 et 200° C, dans une proportion molaire diaminodibenzyle: $P_2O_5$ de 1 : 1 à 1 : 2,5, la neutralisation se produisant alors avec dégagement de chaleur, et en effectuant au cours des stades réactionnels subséquents la condensation à une température de 260 à 320° C, et

b) en transférant alors le mélange réactionnel, non refroidi, en écoulement continu, du dernier stade à un séparateur chauffé et en le séparant à 160 à 300° C en un courant de produit renfermant la phase supérieure d'iminodibenzyle fondu et un courant d'évacuation renfermant, en tant que phase inférieure, de l'acide phosphorique et des phosphates d'ammonium.

2. Procédé suivant la revendication 1, caractérisé en ce qu'on établit au cours du premier stade de réaction une température de 290 à 300° C et l'on maintient, au cours des autres stades de réaction, une température de 280 à 290° C.

3. Procédé suivant les revendication 1 et 2, caractérisé en ce qu'on effectue la réaction en 8 à 15 stades.

4. Procédé suivant les revendications 1 à 3, caractérisé en ce qu'on utilise le 2,2'-diaminodibenzyle et l'acide phosphorique dans une proportion molaire diaminodibenzyle: $P_2O_5$ de 1 : 1 à 1 : 2.

5. Procédé suivant la revendication 4, caractérisé en ce qu'on utilise le 2,2'-diaminodibenzyle et l'acide phosphorique dans une proportion molaire diaminodibenzyle: $P_2O_5$ de 1 : 1 à 1 : 1,5.

6. Procédé suivant les revendications 1 à 5, caractérisé en ce qu'on effectue la séparation en courant de produit et courant d'évacuation à uné température de 230 à 250° C.